# EUROPEAN PATENT APPLICATION

(11) **EP 2 316 344 A1**
(43) Date of publication of application: **04.05.2011**
(21) Application number: 10187085.5
(22) Date of filing: 11.10.2010
(51) Int. Cl.: A61B 8/08, A61B 8/14, G01S 15/89

(54) **Three dimensional pulsed wave spectrum ultrasonic diagnostic apparatus and three dimensional pulsed wave spectrum data generation method**

(30) Priority: 28.10.2009 KR 20090102586
(71) Applicant: MEDISON CO., LTD., Gangwon-do 250-875 (KR)
(72) Inventor: Kim, Beom Gyu, 410-774, Goyang Si Gyeonggi-Do (KR); Yang, Eun Ho, 139-784, Seoul (KR)
(74) Representative: Schmid, Wolfgang

(57) **Abstract**

Disclosed are a three-dimensional (3D) pulsed wave (PW) spectrum ultrasonic diagnostic apparatus and a 3D PW spectrum data generating method. The 3D PW spectrum ultrasonic diagnostic apparatus forms a sample gate in a 3D format by using a two-dimensional (2D) array probe, and generates 3D PW spectrum data by using the formed 3D sample gate.

## Description

### CROSS-REFERENCE TO RELATED APPLICATION

This application claims the benefit of Korean Patent Application No. 10-2009-0102586, filed on Oct. 28, 2009, in the Korean Intellectual Property Office, the disclosure of which is incorporated herein by reference.

### BACKGROUND

### 1. Field

The present invention relates to a three-dimensional (3D) PW spectrum data generating apparatus and method of generating a 3D PW spectrum.

### 2. Description of the Related Art

An ultrasonic system may transmit an ultrasonic signal toward a predetermined portion inside a body from a surface of the body, and may obtain an image of a section of a soft tissue or a bloodstream by using information of an ultrasonic signal reflected from a tissue inside the body. The ultrasonic system has an advantage of being small, inexpensive, and capable of displaying an image in real time. Also, the ultrasonic system has an advantage of being reliable without exposing a subject to an X-ray and the like, and thus, the ultrasonic system is commonly used together with other image diagnostic devices, such as a computerized tomography (CT) scanner, a magnetic resonance image (MRI) device, a nuclear medicine device, and the like.

Accurately measuring a speed of blood flow in a bloodstream is significantly important for a conventional pulsed wave (PW) Doppler scheme. Particularly, in echocardiography, a speed of blood flow in a bloodstream that is beyond a normal range due to coarctation, regurgitation, or the like may be detected by using the conventional PW Doppler, and a speed of the regurgitation is determined and is used as a reference for treatment.

However, when a diagnosis is performed using the conventional PW, a location of a sample gate is often changed due to respiration of a patient or a movement of a sonographer. This may cause repetitious scanning and may hinder an accurate diagnosis.

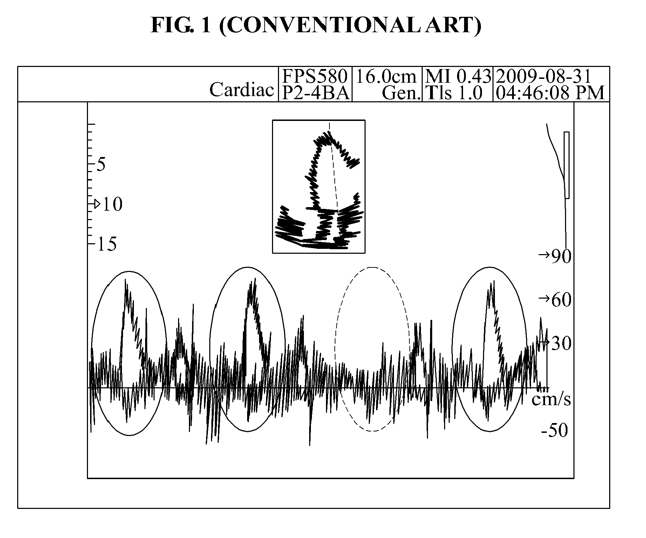

FIG. 1 illustrates an example of a spectrum waveform according to a conventional art. Referring to FIG. 1, a conventional ultrasonic diagnosis system may place a sample gate on a valve of a heart in a PW mode, and may obtain a spectrum waveform. In this instance, a spectrum often does not appear in a location where it was, due to respiration of a patient or a movement of a sonographer. As illustrated in FIG. 1, a third spectrum (inside a dotted circle) disappears due to the respiration of the patient.

Accordingly, there is need for improving inaccuracy and delayed diagnosis due to the respiration of the patient or the movement of the sonographer.

### SUMMARY

An aspect of the present invention provides a 3D PW spectrum ultrasonic diagnostic apparatus and a 3D PW spectrum data generating method that solves a difficulty in diagnosis since a location of a sample gate is changed due to respiration of a patient and a movement of a sonographer, thereby enabling prompt and accurate diagnosis.

According to example embodiments, there may be provided a three-dimensional (3D) pulsed wave (PW) spectrum ultrasonic diagnostic apparatus, the apparatus including a transmitting/receiving unit to transmit a first PW Doppler ultrasonic signal and a second PW Doppler ultrasonic signal to a target object by using a two dimensional (2D) array probe, and to receive a first PW Doppler response signal corresponding to the first PW Doppler ultrasonic signal and a second PW Doppler response signal corresponding to the second PW Doppler ultrasonic signal, a forming unit to form a sample gate in a 3D format by using the received first PW Doppler response signal and the received second PW Doppler response signal, and a generating unit to generate 3D PW spectrum data by using the formed 3D sample gate.

The transmitting/receiving unit may receive a plurality of first PW Doppler response signals related to an X-axis section in response to the first PW Doppler ultrasonic signal, and may receive a plurality of second Doppler response signals related to a Y-axis section in response to the second PW Doppler ultrasonic signal.

The forming unit may form the sample gate having a plurality of sections by using the plurality of first Doppler response signals and the plurality of second Doppler response signals to form a cube sample gate.

The generating unit may generate the 3D PW spectrum data with respect to the target object, an amount of the 3D PW spectrum data being equivalent to a width of the 3D sample gate.

According to example embodiments, there may be provided a method of generating 3D PW spectrum data, the method including transmitting a first PW Doppler ultrasonic signal and a second PW Doppler ultrasonic signal to a target object by using a 2D array probe, receiving, from the target object, a first PW Doppler response signal corresponding to the first PW Doppler ultrasonic signal and a second PW Doppler response signal corresponding to a second PW Doppler ultrasonic signal, forming a sample gate in a 3D format by using the received first PW Doppler response signal and the received second PW Doppler response signal, and generating 3D PW spectrum data by using the formed 3D sample gate.

Additional aspects and/or advantages will be set forth in part in the description which follows and, in part, will be apparent from the description, or may be learned by practice of the embodiments.

### EFFECT

According to the present invention, when a spectrum waveform having a loss due to respiration of a patient or a movement of a sonographer is obtained while a spectrum of an echocardiography is measured, spatial data may be obtained. The spatial spectrum data is providing an effect of obtaining spectrum data with respect to a plurality of sections at a time, an amount of the spectrum data being equivalent to a width of a cube sample gate.

According to an embodiment of the present invention, a 3D PW spectrum includes a spectrum waveform having a loss due to a movement of a patient or a sonographer and a complete spectrum waveform, and thus, the complete spectrum waveform is used as a reference for treatment of the patient, thereby enabling accurate and prompt diagnosis. The complete spectrum waveform is extracted from a plurality of waveforms by using an auto trace and the like, thereby more rapidly obtaining information.

### BRIEF DESCRIPTION OF THE DRAWINGS

These and/or other aspects and advantages will become apparent and more readily appreciated from the following description of the embodiments, taken in conjunction with the accompanying drawings of which:
- FIG. 1: is a diagram illustrating an example of a spectrum waveform according to a conventional art;
- FIG. 2: is a block diagram illustrating a three-dimensional (3D) pulsed wave (PW) spectrum ultrasonic diagnostic apparatus according to an exemplary embodiment of the present invention;
- FIG.3 3: is a diagram illustrating an example of a comparison between a one- dimensional (1D) array probe and a two-dimensional (2D) array of the present invention;
- FIG. 4: is a diagram illustrating an example where a 3D PW spectrum ultrasonic diagnostic apparatus forms a cube sample gate according to an exemplary embodiment of the present invention.; and
- FIG. 5: is a flowchart illustrating a 3D PW spectrum data generating method according to an exemplary embodiment of the present invention.

### DETAILED DESCRIPTION

Reference will now be made in detail to example embodiments, examples of which are illustrated in the accompanying drawings, wherein like reference numerals refer to the like elements throughout. An ultrasonic diagnosis apparatus utilizing a touch interaction is described below to explain the present disclosure by referring to the figures.

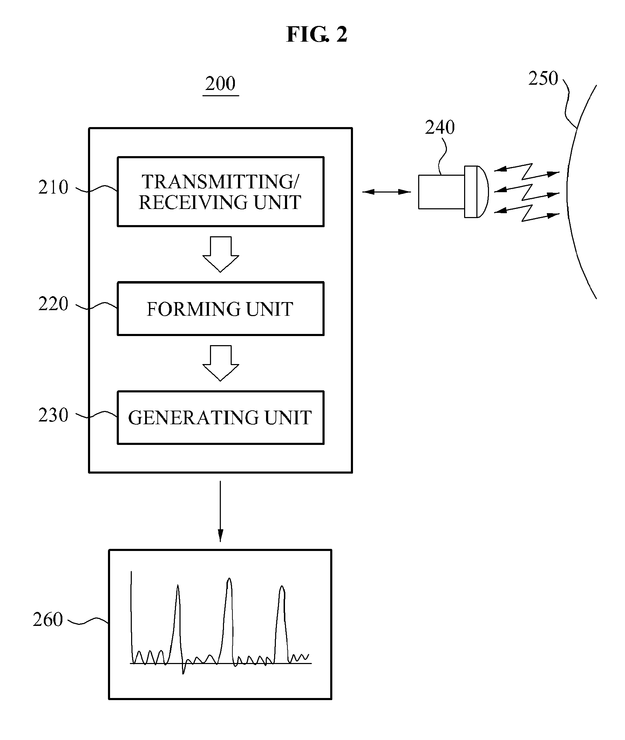

FIG. 2 is a block diagram illustrating a three-dimensional (3D) pulsed wave (PW) spectrum ultrasonic diagnostic apparatus 200.

As illustrated in FIG. 2, the 3D PW spectrum ultrasonic diagnostic apparatus 200 may include a transmitting/receiving unit 210, a forming unit 220, and a generating unit 230.

The transmitting/receiving unit 210 may transmit a first PW Doppler ultrasonic signal and a second PW Doppler ultrasonic signal to a target object 250, and may receive a first PW Doppler response signal corresponding to the first PW Doppler ultrasonic signal and a second PW Doppler response signal corresponding to the second PW Doppler ultrasonic signal, from the target object 250. In this instance, the first PW Doppler response signal and the second PW Doppler response signal are signals that are in response to the first PW Doppler ultrasonic signal and the second PW Doppler ultrasonic signal, respectively, and that may penetrate the target object 250 or may be reflected from the target object 250 Also, the target object 250 may be a body.

According to an embodiment of the present invention, the transmitting/receiving unit 210 may receive a plurality of first PW Doppler response signals related to an X-axis section in response to the first PW Doppler ultrasonic signal, and may receive a plurality of second PW Doppler response signals related to a Y-axis section in response to the second PW Doppler ultrasonic signal.

The forming unit 220 may form a sample gate in a 3D format by using the received plurality of first PW Doppler response signals and the plurality of second PW Doppler response signals. According to an embodiment of the present invention, the forming unit 220 may form the sample gate having a plurality of sections by using the plurality of first PW Doppler response signals and the plurality of second PW Doppler response signals to generate a cube sample gate.

Generally, a spectrum is generated with respect to a single section in a conventional one dimensional (1D) array, and thus, a part of a spectrum waveform may be missing due to respiration of a patient or a movement of a sonographer. To overcome the described weak point, the present invention may receive the plurality of first Doppler response signals and the plurality of second Doppler response signals which enable forming of several or several dozen sections, by using a two-dimensional (2D) array probe 240, thereby minimizing a loss of a part of spectrum waveform due to the respiration of the patient or movement of the sonographer.

The generating unit 230 may generate 3D PW spectrum data by using the formed 3D sample gate. According to embodiment of the present invention, the generating unit 230 may generate 3D PW spectrum data with respect to the target object 250, an amount of the 3D PW spectrum data being equivalent to a width of a cube sample gate that is the 3D sample gate.

As an example, the generated 3D PW spectrum data may include a spectrum waveform having a loss due to the respiration of the patient or the movement of the sonographer and a complete spectrum waveform. Accordingly, the generating unit 230 may extract the complete spectrum waveform from a plurality of spectrum waveforms based on an auto trace and the like. In this instance, the complete spectrum waveform may be used as a reference for treatment of the patient, and thus, the diagnosis is more accurately and rapidly performed.

Accordingly, the 3D PW spectrum data generated by the generating unit 230 may be displayed by a display unit 260.

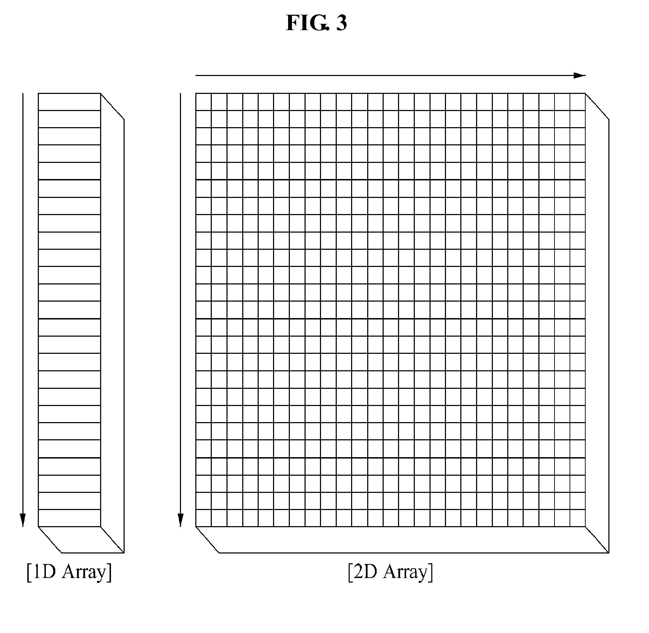

FIG. 3 illustrates an example of a comparison between a conventional 1D array probe and a 2D array of the present invention.

As illustrated in FIG. 3, the conventional 1D array probe (1D Array) may only obtain a spectrum with respect to a single section, whereas the 2D array probe (2D Array) may obtain spectrums with respect to several to several dozen sections by scanning both an X-axis and a Y axis.

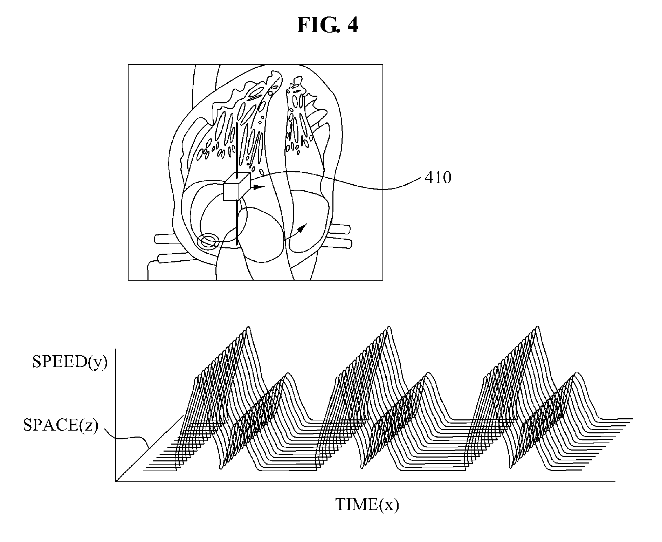

FIG. 4 illustrates an example where a 3D PW spectrum ultrasonic diagnostic apparatus forms a cube sample gate 410 according to an exemplary embodiment of the present invention.

Referring to FIG. 4, the 3D PW spectrum ultrasonic diagnostic apparatus 200 may form the cube sample gate 410 by forming a sample gate having a plurality of sections by using a plurality of first PW Doppler response signals and a plurality of second PW Doppler response signals.

As an example, a valve of a heart is composed of sections. When a width of a sample gate is formed to be equivalent to a width of the cube sample gate 410, a spectrum of the valve that is composed of sections may be observed, a width of the observed spectrum being equivalent to a width of the cube sample gate 410. Accordingly, the 3D PW spectrum ultrasonic diagnostic apparatus 200 may minimize a loss of the spectrum caused by a change in a location of the sample gate due to respiration of a patient.

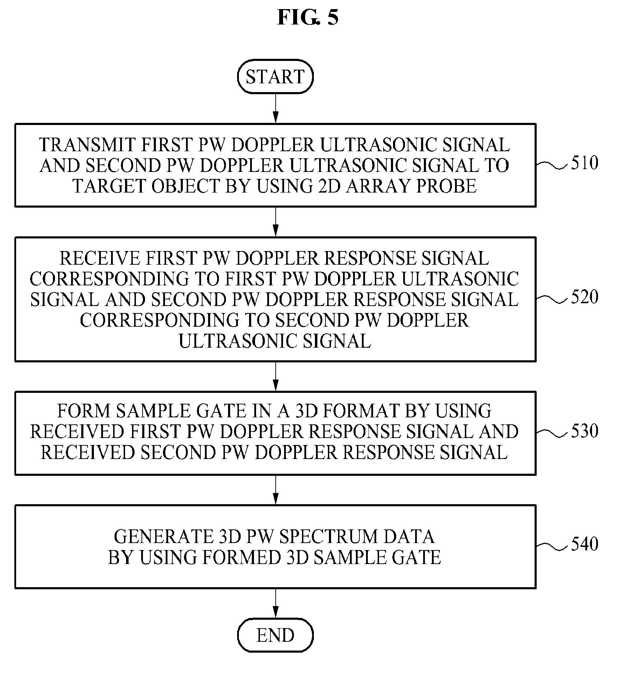

FIG. 5 is a flowchart illustrating a 3D PW spectrum data generating method according to an exemplary embodiment of the present invention.

In operation 510, a 3D PW spectrum data generating method transmits a first PW Doppler ultrasonic signal and a second PW Doppler ultrasonic signal to a target object by using the 2D array probe 240.

In operation 520, the 3D PW spectrum data generating method receives a first PW Doppler response signal corresponding to the first PW Doppler ultrasonic signal and a second PW Doppler response signal corresponding to the second PW Doppler ultrasonic signal, from the target object 250.

In operation 530, the 3D PW spectrum data generating method forms a sample gate in a 3D format by using the received first PW Doppler response signal and the second PW Doppler response signal.

In operation 540, the 3D PW spectrum data generating method generates 3D PW spectrum data by using the formed 3D sample gate.

The descriptions of the 3D PW spectrum ultrasonic diagnostic apparatus 200 described with respect to FIG. 2 to FIG. 4 may be applied to the 3D PW spectrum data generating method illustrated in FIG. 5. Accordingly, further description of the 3D PW spectrum data generating method will be omitted.

The method according to the above-described exemplary embodiments of the present invention may be recorded in computer-readable media including program instructions to implement various operations embodied by a computer. The media may also include, alone or in combination with the program instructions, data files, data structures, and the like. Examples of computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few example embodiments have been shown and described, it would be appreciated by those skilled in the art that changes may be made in these example embodiments without departing from the principles and spirit of the invention, the scope of which is defined in the claims and their equivalents.

## Claims

1. A three-dimensional (3D) pulsed wave (PW) spectrum ultrasonic diagnostic apparatus, the apparatus comprising:
a transmitting/receiving unit to transmit a first PW Doppler ultrasonic signal and a second PW Doppler ultrasonic signal to a target object by using a two dimensional (2D) array probe, and to receive a first PW Doppler response signal corresponding to the first PW Doppler ultrasonic signal and a second PW Doppler response signal corresponding to the second PW Doppler ultrasonic signal;
a forming unit to form a sample gate in a 3D format by using the received first PW Doppler response signal and the received second PW Doppler response signal; and
a generating unit to generate 3D PW spectrum data by using the formed 3D sample gate.

2. The apparatus of claim 1, wherein the transmitting/receiving unit receives a plurality of first PW Doppler response signals related to an X-axis section in response to the first PW Doppler ultrasonic signal, and receives a plurality of second Doppler response signals related to a Y-axis section in response to the second PW Doppler ultrasonic signal.

3. The apparatus of claim 2, wherein the forming unit forms the sample gate having a plurality of sections by using the plurality of first Doppler response signals and the plurality of second Doppler response signals to form a cube sample gate.

4. The apparatus of claim 1, wherein the generating unit generates the 3D PW spectrum data with respect to the target object, an amount of the 3D PW spectrum data being equivalent to a width of the 3D sample gate.

5. A method of generating 3D PW spectrum data, the method comprising:
transmitting a first PW Doppler ultrasonic signal and a second PW Doppler ultrasonic signal to a target object by using a 2D array probe;
receiving, from the target object, a first PW Doppler response signal corresponding to the first PW Doppler ultrasonic signal and a second PW Doppler response signal corresponding to a second PW Doppler ultrasonic signal;
forming a sample gate in a 3D format by using the received first PW Doppler response signal and the received second PW Doppler response signal; and
generating 3D PW spectrum data by using the formed 3D sample gate.

6. The method of claim 5, wherein the receiving comprises:
receiving a plurality of first PW Doppler response signals related to an X-axis section in response to the first PW Doppler ultrasonic signal; and
receiving a plurality of second PW Doppler response signals related to a Y-axis section in response to the second PW Doppler ultrasonic signal.

7. The method of claim 6, wherein:
the forming comprises:
forming a cube sample gate by forming the sample gate having a plurality of sections by using the plurality of first PW Doppler response signals and the plurality of second PW Doppler response signals, and
the generating comprises:
generating the 3D PW spectrum data with respect to the target object, an amount of the 3D PW spectrum data being equivalent to a width of the 3D sample gate.
